# EUROPEAN PATENT SPECIFICATION

(11) **EP 3 574 112 B1**
(45) Date of publication and mention of the grant of the patent: **11.01.2023**
(21) Application number: 18702220.7
(22) Date of filing: 26.01.2018
(51) Int. Cl.: C12Q 1/686, C12Q 1/6869, C12Q 1/6813

(54) **BARCODED DNA FOR LONG RANGE SEQUENCING**
STRICHCODIERTE DNS FÜR LANGE SEQUENZIERUNG
ADN A CODE-BARRES POUR LE SEQUENÇAGE LONG

(30) Priority: 27.01.2017 US 201762451354 P
(43) Date of publication of application: 04.12.2019
(73) Proprietor: Roche Diagnostics GmbH, 68305 Mannheim (DE); F. Hoffmann-La Roche AG, 4070 Basel (CH)
(72) Inventor: NOLAN, Garry, Pleasanton, CA 94588 (US)
(74) Representative: Hildebrandt, Martin K. E.
(86) International application number: PCT/EP2018/051909
(87) International publication number: WO 2018/138237

(56) References cited:
- WO-A1-2016/081740
- WO-A1-2016/149418
- WO-A2-2012/106385
- WO-A2-2016/100976

## Description

### BACKGROUND

This invention pertains to the field of DNA sequencing, and in particular, methods for facilitating sequence assembly and phased sequencing.

### SUMMARY

The invention provides methods for facilitating nucleic acid sequence assembly. The method comprises the steps of a) providing a nucleic acid sample comprising single-stranded template nucleic acids; b) priming each template nucleic acid with one or more barcode initiation primers to create annealed nucleic acid assemblies, wherein each barcode initiation primer comprises a 3'- template hybridization portion, and a 5'- barcode initiation portion; c) performing a polymerase extension to create template complementary strands which remain annealed to the template nucleic acids; d) performing two or more rounds of split-pool synthesis coupling oligonucleotide coding units "assayable polymer subunits" (APS) to assemble an oligonucleotide barcode sequence on each of the template complementary strands comprising the barcode region, the primer region and the target-specific region complementary to the target nucleic acid; e) sequencing the barcoded complementary strands to obtain template DNA sequence reads; and f) identifying the barcode sequence associated with each template DNA sequence read to identify those sequences that arose from the same template nucleic acid and assembling the template nucleic acid sequence from the template-complementary regions of the barcoded complementary strands.

ln some embodiments, the method further comprises end-labeling the single-stranded template nucleic acids at the 3'-end with an affinity tag prior to step (b). In some embodiments, the affinity tag is used to purify the barcoded strands of step (e) prior to performing step (e). In some embodiments, the affinity tag is biotin. In some embodiments, the nucleic acid is DNA. In some embodiments, the DNA is genomic DNA or exomic DNA. In some embodiments, the first sequence region of the barcode initiation primer molecules comprises a random primer sequence region. In some embodiments, the random primer sequence region is from about 4 nucleotides to about 10 nucleotides in length. In some embodiments, the first sequence region of the barcode initiation primer molecules comprises a semi-random primer sequence region. In some embodiments, the semi-random primer sequence region is from about 4 nucleotides to about 10 nucleotides in length. In some embodiments, the barcode initiation primer molecules further comprise an amplification primer binding sequence, a sequencing primer binding sequence, or both. In some embodiments, the barcode initiation primers bind to the template nucleic acids every 50 - 1,000 base pairs on average. In some embodiments, the polymerase extension reaction further comprises the use of a dideoxy-nucleotide to introduce synthesis stops, thereby preventing or minimizing displacement of polymerase molecules from the annealed nucleic acid assemblies. In some embodiments, the APS are selected from a pool of from 2 to 200 unique APS. In some embodiments, the APS are oligonucleotides. In some embodiments, the APS further comprise error checking sub-codes. In some embodiments, each APS further comprises a random or semi-random tag sequence. In some embodiments, the random or semi-random tag sequence is from 2 to 8 nucleotides in length. In some embodiments, the random or semi-random tag sequences associated with each individual barcoded complementary nucleic acid copy of a template nucleic acid fragment function as a unique molecular counter sequence. In some embodiments, the number of different molecular counter sequences associated with a template nucleic acid barcode sequence is used to enumerate the initial number of complementary DNA copies that were derived from a given template DNA nucleic acid, or to detect repeat sequences within a given template DNA nucleic acid. In some embodiments, the molecular counter sequences are used to determine the probability that a template nucleic acid sequence variant was derived from a polymerase error during an amplification reaction. In some embodiments, the APS are assembled during the two or more rounds of split-pool synthesis by annealing to a splint molecule. In some embodiments, the APS are assembled during the two or more rounds of split pool synthesis using ligation or Click chemistry. In some embodiments, the method is used for long-range DNA sequence assembly. In some embodiments, the method is used for sequence phasing. In some embodiments, the method is used for haplotyping. In some embodiments, the method is used for determination of genomic DNA structural variations selected from the group consisting of deletions, insertions, duplications, translocations, and inversions.

In some embodiments, the barcode initiation primers comprise random primer sequence regions. In some embodiments, the barcode initiation primers comprise semi-random primer sequence regions. In some embodiments, the set of APS comprise from 2 to 20 unique APS. In some embodiments, the APS are oligonucleotides.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

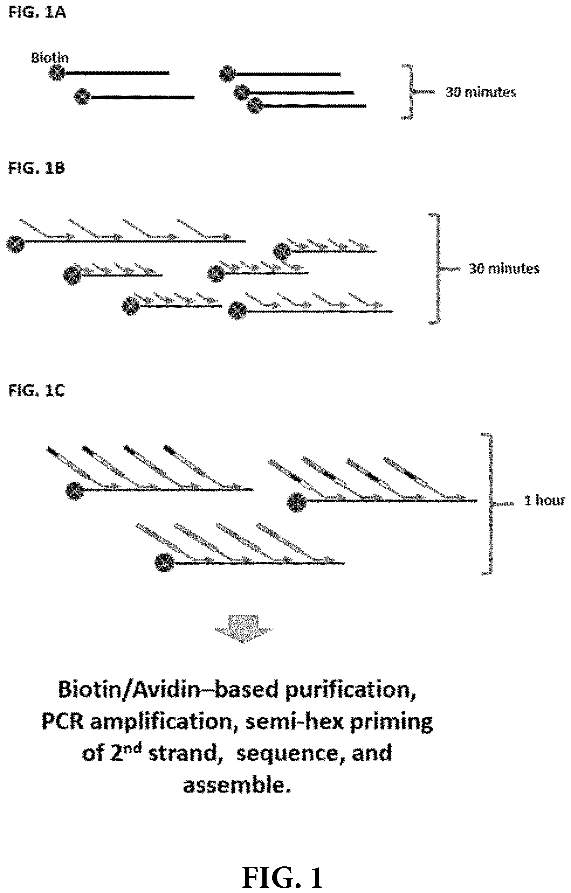

FIGS. 1 A-C illustrate one embodiment of the methods disclosed herein, whereby a plurality of barcoded DNA nucleic acids are produced for each individual template DNA nucleic acid in a sample.

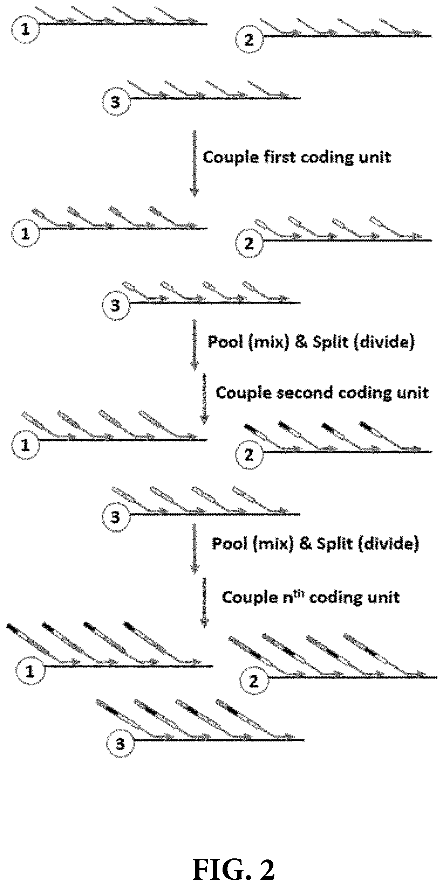

FIG. 2 illustrates one embodiment of a split-pool synthesis approach for adding barcodes to each of the complementary DNA sequences arising from a plurality of primer extension reactions, wherein the barcodes code for the identity of the individual template DNA nucleic acid from which each individual strand of complementary DNA was synthesized.

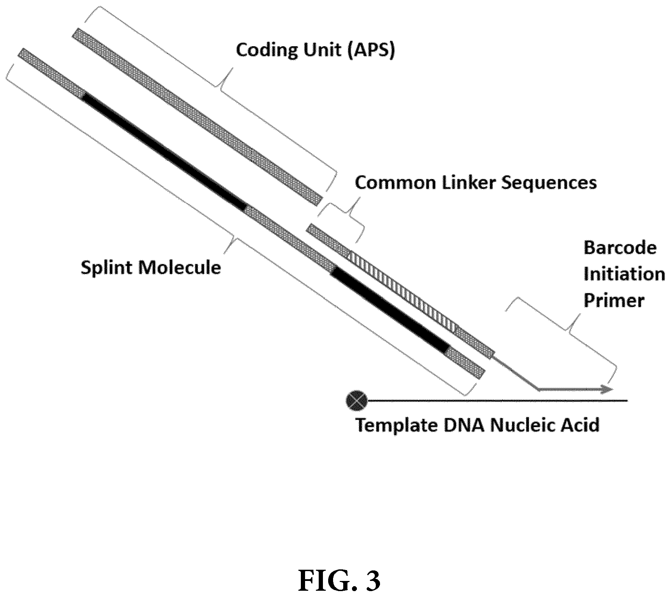

FIG. 3 provides a schematic illustration of one non-limiting method for assembling and synthesizing a template nucleic acid barcode using a "splint" molecule comprising common linker sequences to assemble a series of coding units (APS) comprising complementary common linker sequences. Several rounds of split-pool synthesis, each comprising annealing and ligation of a different coding unit for each sample pool in each round, may be used to create template nucleic acid barcodes.

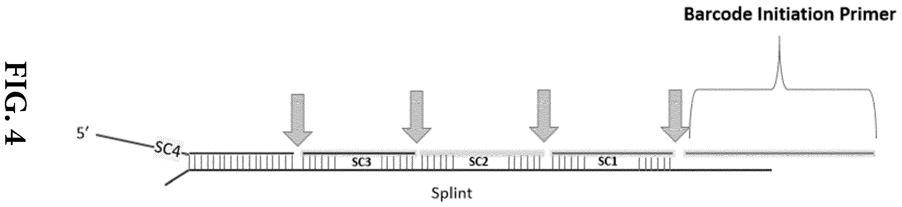

FIG. 4 shows a schematic illustration of one embodiment of a molecular complex comprising a barcode initiation primer, a splint molecule, and a template DNA nucleic acid barcode comprising 4 APS coding units (further comprising sub-codes SCI - SC4). Each APS code comprises a sub-code region flanked on either end by annealing sequences (common linker sequences) that are complementary to a splint oligonucleotide, which is itself annealed to a complementary region of the barcode initiation primer. Following the combinatorial assembly of the template DNA nucleic acid barcode by means of annealing to the splint oligonucleotide, the APS subunits are ligated (at positions indicated by arrows) to form a single, covalently-linked molecular complex.

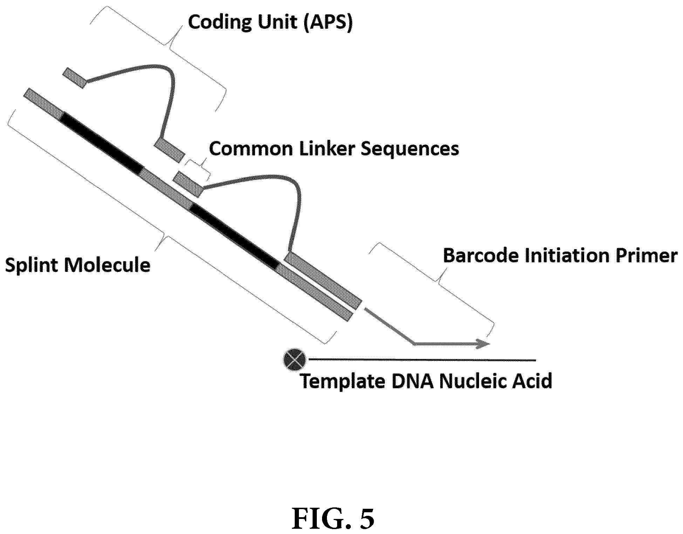

FIG. 5 provides a schematic illustration of one non-limiting method for assembling and synthesizing a template nucleic acid barcode using a "splint" molecule comprising common linker sequences to assemble a series of coding units (APS) comprising complementary loop-forming common linker sequences. Several rounds of split-pool synthesis, each comprising annealing and ligation of a different coding unit for each sample pool in each round, may be used to create template nucleic acid barcodes.

### DETAILED DESCRIPTION

Disclosed herein but not part of the invention are methods for performing template DNA barcoding for use in facilitating DNA sequence assembly and phased sequencing. In some instances, the methods comprise fragmenting DNA that has been extracted from a sample; adding an affinity tag to at least a first end of the template DNA; hybridizing at least a first barcode initiation primer to the tagged template DNA nucleic acid, wherein the at least first barcode initiation primer comprises at least a first sequence region that hybridizes to the template DNA nucleic acid and a second sequence region that further comprises a barcode initiation site, *e.g.,* a barcode initiator sequence, a barcode initiator moiety, or a common linker sequence for hybridizing to a "splint" molecule; performing a polymerase extension reaction to create at least a first strand of complementary DNA that is annealed to all or a portion of the template DNA nucleic acid, and subsequently performing successive rounds of split-pool synthesis to add two or more coding units to each annealed strand of complementary DNA to create a unique barcode that serves to identify the individual template DNA fragment from which the at least a first strand of complementary DNA was synthesized (FIGS. 1 A-C). Advantageously, the disclosed DNA barcoding methods do not require partitioning of the individual template DNA nucleic acids into individual compartments prior to barcoding, amplification, and sequencing. Also disclosed herein are kits comprising the reagents required to perform the DNA barcoding methods, and systems configured to perform the DNA barcoding methods. used to facilitate sequence assembly, *e.g*., genomic DNA sequence assembly. For example, in some instances, a plurality (or "set") of barcoded, complementary DNA sequences may be prepared from each individual template DNA nucleic acid by priming at multiple sites along the template DNA nucleic acid, performing a polymerase extension reaction for each of the annealed primers to synthesize DNA that is complementary to at least a portion of the template DNA nucleic acid, and subsequently using a split-pool synthesis approach to add two or more coding units to each of the annealed primer-complement sequences to create the unique barcodes that code for the identities of the individual template DNA nucleic acids from which each set of barcoded, complementary DNA sequences was prepared. Following amplification and sequencing of the barcoded, complementary DNA sequences, the barcode sequence region for each sequence read may be used to identify and align the set of complementary DNA sequences for each template DNA nucleic acid, thereby enabling efficient long-range sequence assembly and sequence determination for DNA nucleic acids that are much larger than the typical read length for modern, high-throughput sequencing technologies.

The methods of the invention are used to facilitate phased sequencing, assembly of long DNA strands containing repetitive sequences, resolution of chromosomal inversions, deletions, or insertions, overcoming the limitations of short sequence reads, and identification of haplotypes. For instance, short sequence reads are unable to resolve important chromosomal differences in cancer such as inversions, translocations, or duplications. For these, long sequence reads that span the duplicated region, or inversions /translocations are required to correctly "place" the DNA in the context of its correct chromosomal position. For haplotype determination, whole-genome sequencing typically generates a single consensus sequence without distinguishing between the sequence variants, *e.g.,* single nucleotide polymorphisms (SNPs), insertions, deletions, and/or mutations, found on different homologous chromosomes, *e.g.,* those derived from the maternal and paternal lines of inheritance. Phased sequencing addresses this limitation by determining which genetic variants appear on the same or different chromosomes and are thus inherited together or separately. Information about patterns of genetic variation, such as haplotype (i.e., a group of sequence variants that are inherited as a contiguous block), is important in understanding genetically-inherited traits and genetic disease, as is information about the copy number of specific genes. Phased sequencing provides an approach for determining haplotype as well as identifying the presence of de novo mutations, and thus has applications in studies of population genetics and genetic disease.

In some instances, the barcode sequences created using the disclosed methods may be used to identify those DNA sequences (or complements thereof) that are associated with the same DNA strand and/or the same chromosome without the need for partitioning individual DNA strands or chromosomes into separate compartments prior to performing amplification and sequencing reactions. In some instances, the barcode information may be used in conjunction with one or more known marker gene sequences to identify those DNA sequences (or complements thereof) that are associated with the same DNA strand and/or the same chromosome without the need for partitioning individual DNA strands or chromosomes into separate compartments prior to performing amplification and sequencing reactions. In some instances, conclusions regarding the phasing of particular genetic variants may then be drawn. Such information may be useful for identifying haplotypes, *i.e.,* a specified set of genetic variants that reside on the same nucleic acid strand or on different nucleic acid strands. In some instances, determination of the number of unique barcodes associated with one or more specified known gene sequences may be used to identify copy number variations for those genes.

*Definitions:* Unless otherwise defined, all technical terms used herein have the same meaning as commonly understood by one of ordinary skill in the art in the field to which this disclosure belongs. As used in this specification and the appended claims, the singular forms "a", "an", and "the" include plural references unless the context clearly dictates otherwise. Any reference to "or" herein is intended to encompass "and/or" unless otherwise stated.

*Samples:* The methods may be used for the analysis of DNA (or other nucleic acid molecules) extracted from any of a variety of samples including, but not limited to, blood, cells, cell suspensions, sub-cellular organelles, tissue samples, and the like.

*Nucleic acids:* The term "nucleic acid" refers to a nucleotide polymer or fragments therof, and unless otherwise limited, includes known analogs of natural nucleotides that can function in a similar manner (e.g., hybridize) to naturally occurring nucleotides. Methods be used for the analysis of DNA or nucleic acid molecules other than DNA including, but not limited to, genomic DNA, chromosomal DNA, mitochondrial DNA, RNA, messenger RNA (mRNA), transfer RNA (tRNA), or complementary DNA (cDNA) synthesized by reverse transcription of RNA, mRNA, or tRNA, and the like.

*DNA extraction and fragmentation:* DNA extraction from a variety of biological samples may be performed using any number of techniques known to those of skill in the art. A typical DNA extraction procedure comprises (i) collection of the cell sample or tissue sample from which DNA is to be extracted, (ii) disruption of cell membranes *(i.e.,* cell lysis) to release DNA and other cytoplasmic components, (iii) treatment of the lysed sample with a concentrated salt solution to precipitate proteins, lipids, and RNA, followed by centrifugation to separate out the precipitated proteins, lipids, and RNA, and (iv) purification of DNA from the supernatant to remove detergents, proteins, salts, or other reagents used during the cell membrane lysis step.

Disruption of cell membranes may be performed using a variety of mechanical shear (e.g., by passing through a French press or fine needle) or ultrasonic disruption techniques. The cell lysis step often comprises the use of detergents and surfactants to solubilize lipids the cellular and nuclear membranes. In some instances, the lysis step may further comprise use of proteases to break down protein, and/or the use of an RNase for digestion of RNA in the sample.

Examples of suitable techniques for DNA purification include, but are not limited to, (i) precipitation in ice-cold ethanol or isopropanol, followed by centrifugation (precipitation of DNA may be enhanced by increasing ionic strength, e.g., by addition of sodium acetate), (ii) phenol- chloroform extraction, followed by centrifugation to separate the aqueous phase containing the nucleic acid from the organic phase containing denatured protein, and (iii) solid phase chromatography where the nucleic acids adsorb to the solid phase (e.g., silica or other) depending on the pH and salt concentration of the buffer.

In some instances, cellular and histone proteins bound to the DNA may be removed either by adding a protease or by having precipitated the proteins with sodium or ammonium acetate, or through extraction with a phenol-chloroform mixture prior to a DNA precipitation step.

In some instances, DNA may be extracted using any of a variety of suitable commercial DNA extraction and purification kits. Examples include, but are not limited to, the QIAamp (for isolation of genomic DNA from human samples) and DNAeasy (for isolation of genomic DNA from animal or plant samples) kits from Qiagen (Germantown, MD) or the Maxwell^{®} and ReliaPrep^{™} series of kits from Promega (Madison, WI).

After isolation, the DNA is typically dissolved in a slightly alkaline buffer, *e.g.,* Tris-EDTA (TE) buffer, or in ultra-pure water. Additional DNA fragmentation, if necessary, may be performed using mechanical fragmentation *(e.g.,* using sonication, needle shear, nebulization, point-sink shearing, or passage through a pressure cell) or enzymatic digestion techniques *(e.g.,* with the use of restriction enzymes or endonucleases).

The template DNA nucleic acids or fragments suitable for use in the disclosed DNA barcoding methods may be substantially longer than those typically used with current high-throughput sequencing technologies. In some instances, the average length of the template DNA nucleic acids may range from about 25 bases (nucleotides for single-stranded DNA, or base pairs for double-stranded DNA) to about 100 kilobases. In some instances, the average length of template DNA nucleic acids may be at least 25 bases, at least 50 bases, at least 75 bases, at least 100 bases, at least 250 bases, at least 500 bases, at least 750 bases, at least 1,000 bases, at least 10 kilobases, at least 20 kilobases, at least 30 kilobases, at least 40 kilobases, at least 50 kilobases, at least 60 kilobases, at least 70 kilobases, at least 80 kilobases, at least 90 kilobases, or at least 100 kilobases. In some instances, the average length of the template DNA fragments may be at most 100 kilobases, at most 90 kilobases, at most 80 kilobases, at most 70 kilobases, at most 60 kilobases, at most 50 kilobases, at most 40 kilobases, at most 30 kilobases, at most 20 kilobases, at most 10 kilobases, at most 1,000 bases, at most 750 bases, at most 500 bases, at most 250 bases, at most 100 bases, at most 75 bases, at most 50 bases, or at most 25 bases. Any of the lower and upper values described in this paragraph maybe combined to form a range included within the disclosure, for example, the average length of the template DNA fragments may range from about 750 bases to about 20 kilobases. Those of skill in the art will recognize that the average length of the template DNA fragments may have any value within this range, e.g., about 4.5 kilobases.

*Affinity tags for purification of template DNA fragments and associated complementary strands:* In some instances, template DNA nucleic acids may be labeled with an affinity tag to facilitate subsequent separation and purification steps. Any of a variety of affinity tags known to those of skill in the art may be used. For example, in some instances, the 3'-end of denatured (single stranded) template DNA may be labeled with a biotin moiety to facilitate separation and purification steps using avidin or streptavidin capture techniques. 3'-labeling of denatured (single-stranded) DNA may be accomplished using a variety of techniques known to those of skill in the art. Examples include, but are not limited to, non-template directed nucleotide incorporation at the 3'-OH end of single-stranded DNA by terminal deoxynucleotidyl transferase (TdT, or "terminal transferase"). TdT exhibits a strong preference for single-stranded DNA, but will also label duplex DNA with 3' overhangs and blunt duplexes with a lower efficiency. TdT is useful for incorporation of modified nucleotides, e.g., biotinylated nucleotides, at the 3'-end of single-stranded DNA. Labeling of single-stranded DNA nucleic acids at the 3'-end with biotin may be performed using commercial kits such as the Pierce - Biotin 3' End DNA Labeling Kit, Catalog #89818 (ThermoFisher Scientific, Waltham, MA).

Biotinylated molecules, *e.g.,* biotinylated DNA nucleic acids may be affinity purified using any of a number of techniques known to those of skill in the art. Typically, the biotinylated species is passed over a resin or bead-based matrix comprising an immobilized avidin or streptavidin protein, or subdomains thereof, and the strong noncovalent interaction between the biotin moiety and the avidin or streptavidin binding pocket permits separation of the biotinylated species from other components of a reaction mixture. Some biotin analogues, *e.g.,* desthiobiotin, bind reversibly to avidin-like proteins and allow elution of biotinylated molecules from avidin-like proteins using less harsh elution conditions.

Affinity purification resins and other affinity-based capture products for the purification of biotinylated or desthiobiotinylated proteins, peptides, oligonucleotides, or other molecules typically comprise avidin, streptavidin, NeutrAvidin^{™}, or CaptAvidin^{™} proteins or subdomains thereof, and are available in a variety of formats including, but not limited to, bead-based resins, coated magnetic beads, spin columns, coated microplates, and ligand-specific kits. CaptAvidin^{™} (ThermoFisher Scientific, Catalog No. C21 386) is a biotin-binding protein that contains a nitrated tyrosine in its biotin-binding site. This chemical modification also permits dissociation of the avidin-biotin complex under milder conditions than the conventional avidin-biotin complex.

*Barcode initiation primers for synthesis of complementary DNA strands:* Complementary DNA strands that comprise complementary sequences to all or a portion of the template DNA fragments may be synthesized by performing primer extension reactions. In some instances, a plurality of barcode initiation primers may be hybridized to each template DNA *(i.e.,* at multiple sites along the template DNA) under suitable hybridization conditions, and a plurality (or "set") of barcoded complementary DNA sequences or strands may be prepared by performing a polymerase extension reaction for each of the annealed primers to synthesize DNA that is complementary to all or a portion of the template DNA nucleic acid (FIGS. 1 A-C).

In some instances, the average number of barcode initiation primers bound to each template DNA nucleic acid under suitable annealing conditions may vary depending upon need. In some instances, barcode initiation primers may bind to template DNA nucleic acids every 50 base pairs to every 10,000 base pairs on average. More frequent binding facilitates more complete sequencing coverage of the genome. In some instances, barcode initiation primers may bind to template DNA nucleic acids at least every 50 base pairs, at least every 100 base pairs, at least every 500 base pairs, at least every 1,000 base pairs, at least every 2,000 base pairs, at least every 3,000 base pairs, at least every 4,000 base pairs, at least every 5,000 base pairs, at least every 6,000 base pairs, at least every 7,000 base pairs, at least every 8,000 base pairs, at least every 9,000 base pairs, or at least every 10,000 base pairs on average. In some instances, barcode initiation primers may bind to template DNA nucleic acids at most every 10,000 base pairs, at most every 9,000 base pairs, at most every 8,000 base pairs, at most every 7,000 base pairs, at most every 6,000 base pairs, at most every 5,000 base pairs, at most every 4,000 base pairs, at most every 3,000 base pairs, at most every 2,000 base pairs, at most every 1,000 base pairs, at most every 500 base pairs, at most every 100 base pairs, or at most every 50 base pairs on average. Any of the lower and upper values described in this paragraph may be combined to form a range included within the disclosure, for example, barcode initiation primers may bind to template DNA nucleic acid sequences every 500 base pairs to every 4,000 base pairs on average. Those of skill in the art will recognize that barcode initiation primers may bind to template DNA nucleic acids with a frequency having any value within this range, *e.g.,* about every 5,250 base pairs on average.

In some instances, the average number of barcode initiation primers bound to each template DNA nucleic acid may be at least 1, at least 2, at least 3, at least 4, at least 5, at least 1 0, at least 15, at least 20, at least 25, at least 30, at least 35, at least 40, at least 45, or at least 50. In some instances, the average number of barcode initiation primers bound to each template DNA nucleic acid may be at most 50, at most 45, at most 40, at most 35, at most 30, at most 25, at most 20, at most 15, at most 10, at most 5, at most 4, at most 3, at most 2, or at most 1. Any of the lower and upper values described in this paragraph may be combined to form a range included within the disclosure, for example, the average number of barcode initiation primers bound to each template DNA nucleic acid may range from about 5 to about 30. Those of skill in the art will recognize that the average number of barcode initiation primer nucleic acids bound to each template DNA nucleic acid may have any value within this range, e.g., about 12.

In general, the barcode initiation primers used in the disclosed DNA barcoding methods may comprise a first sequence region that is capable of hybridizing to the template DNA fragment, and a second sequence region that comprises a barcode initiation site, *e.g.,* a barcode initiator sequence, a barcode initiator moiety, or a common linker sequence for hybridizing to a "splint" molecule. In some instances, the first sequence region may comprise random, semi-random, or target specific sequences capable of hybridizing to the template DNA nucleic acid. In some instances, the barcode initiation primers used in the disclosed DNA barcoding methods may further comprise at least a third sequence region, wherein the at least third sequence region may comprise a linker sequence for attaching a detectable label, a spacer sequence used to separate the first sequence region and the second sequence region, an amplification primer binding site, a sequencing primer binding site, or any combination thereof.

*Random priming:* In some instances, polymerase extension reactions to synthesize the complementary strand of single-stranded template DNA may be performed using a random priming approach, *e.g.,* using short oligonucleotide regions of random sequence *(i.e.,* "random barcode initiation primers") for the first sequence region of the barcode initiation primer that recognize and bind to the template DNA at arbitrary positions, thereby allowing a suitable DNA polymerase *(e.g.,* the Klenow polymerase) to incorporate nucleotides at the 3'-OH terminus of the annealed random primers. In some instances, the length of the random primer sequence region may range from about 4 nucleotides to about 12 nucleotides, *i.e.,* long enough to provide stable hybridization at the annealing temperature and short enough that the primers will recognize and bind to the template DNA at multiple sites. In some instances, the length of the random primer sequence region may be at least 4 nucleotides, at least 6 nucleotides, at least 8 nucleotides, at least 10 nucleotides, or at least 12 nucleotides. In some instances, the length of the random primer sequence region may be at most 12 nucleotides, at most 10 nucleotides, at most 8 nucleotides, at most 6 nucleotides, or at most 4 nucleotides. The number of nucleotides used or required may vary with the temperature of annealing and/or solvent conditions that affect the melting temperate of the annealed product. Any of the lower and upper values described in this paragraph may be combined to form a range included within the disclosure, for example, the length of the random primer sequence region may range from about 4 nucleotides to about 8 nucleotides. Those of skill in the art will recognize that the length of the random primer sequence region may have any value within this range, *e.g.,* about 7 nucleotides.

In some instances, the random barcode initiation primer may comprise a random first sequence region *(e.g.,* "NNNNNN") located near the 3'-end of the molecule, where NNNNNN is a random six nucleotide sequence. In some instances, the random barcode initiation primer may comprise an amplification primer binding site and/or a sequencing primer region *(e.g.,* an Illumina sequencing primer sequence) located near the 5'-end of the molecule. As indicated above, the random primers may further comprise a second sequence region that further comprises a barcode initiation site, *e.g.,* a barcode initiator sequence, a barcode initiator moiety, or a common linker sequence for hybridizing to a "splint" molecule, located near the 5'-end of the molecule. In some instances, the random first sequence region, the amplification and/or sequencing primer binding region, and/or the second sequence region may be separated by one or more spacer sequences.

*Semi-random priming:* In some instances, polymerase extension reactions to synthesize the complementary strand of single-stranded template DNA may be performed using a semi-random priming approach, e.g., using short oligonucleotides comprising a semi-random sequence *(i.e.,* "semi-random barcode initiation primers") for the first sequence region of the barcode initiation primer that recognize and bind to the template DNA at semi-arbitrary positions with an adjustable frequency, thereby allowing a suitable DNA polymerase *(e .g.,* the Klenow polymerase) to incorporate nucleotides at the 3'-OH terminus of the annealed semi-random primers. In some instances, the length of the semi-random primer sequence region may range from about 4 nucleotides to about 12 nucleotides, *i.e.,* long enough to provide stable hybridization at the annealing temperature and short enough that the primers will recognize and bind to the template DNA at multiple sites. In some instances, the length of the semi-random primer sequence region may be at least 4 nucleotides, at least 6 nucleotides, at least 8 nucleotides, at least 10 nucleotides, or at least 12 nucleotides. In some instances, the length of the semi-random primer sequence region may be at most 12 nucleotides, at most 10 nucleotides, at most 8 nucleotides, at most 6 nucleotides, or at most 4 nucleotides. Any of the lower and upper values described in this paragraph may be combined to form a range included within the disclosure, for example, the length of the semi-random primer sequence region may range from about 4 nucleotides to about 8 nucleotides. Those of skill in the art will recognize that the length of the semi-random primer sequence region may have any value within this range, e.g., about 7 nucleotides.

In some instances, the length of the non-random portion of the semi-random primer sequence may range from about 2 to about 4 nucleotides in length. The choice of non-random nucleotide sequence used will determine how often the semi-random primer can bind to the template DNA on average. For example, the sequence XXXCCC (where X is a random nucleotide) will bind, on average, once every 64 base pairs depending on the GC content of the template DNA.

In some instances, the semi-random barcode initiation primer may comprise a semi-random first sequence region *(e.g.,* "NNNGAG") located near the 3'-end of the molecule, where NNN is a random three nucleotide sequence. In some instances, the semi-random barcode initiation primer may comprise an amplification primer binding site and/or a sequencing primer region *(e.g.,* an Illumina sequencing primer sequence) located near the 5'-end of the molecule. As indicated above, the semi-random barcode initiation primers may further comprise a second sequence region that further comprises a barcode initiation site, *e.g.,* a barcode initiator sequence, a barcode initiator moiety, or a common linker sequence for hybridizing to a "splint" molecule, located near the 5'-end of the molecule. In some instances, the semi-random first sequence region, the amplification and/or sequencing primer binding region, and/or the second sequence region may be separated by one or more spacer sequences.

In some instances, the semi-random sequence region may be of the form of (M)ᵢ(X)ⱼ(N)ₖ, where (M)i and (N)ₖ are any random nucleotide sequences of length i and k respectively, and where (X)j is a specific oligonucleotide sequence of length j chosen to complement the template DNA nucleic acid sequence at a series of specified sub-sequence position(s). Typically, the value of i and k may range between 0 and 6, and the value of j may range between 3 and 6. In some instances, the semi-random sequence region may be designed to be complementary to a specified template DNA nucleic acid sequence at a specified position relative to the 3'-end of a known or partially known template DNA nucleic acid sequence, thereby yielding an amplification product that is approximately Z nucleotides in length, where the value of Z may typically range from 50 to 1000.

*Barcode initiation sequence region:* As indicated above, in some instances a plurality of barcode initiation primers may be hybridized to each template DNA fragment *(i.e.,* at multiple sites along the template DNA nucleic acid) under suitable hybridization conditions, and a plurality (or "set" ) of barcoded, complementary DNA sequences may be subsequently synthesized by first performing a polymerase extension reaction on each of the annealed primers, and then performing multiple rounds of split-pool synthesis to create a barcode that identifies the individual template DNA nucleic acid from which the set of barcoded, complementary DNA sequences arose (FIGS. 1 A-C and 2). The creation of the barcode requires that the plurality of barcode initiation primers comprise a second sequence region that further comprises a barcode initiation site, *e.g.,* a barcode initiator sequence, a barcode initiator moiety, or a common linker sequence for hybridizing to a "splint" molecule. Any of a variety of barcode synthesis initiation techniques known to those of skill in the art may be used. In some instances, for example, a barcode initiator sequence may comprise a 5'-azide terminal nucleotide for subsequent use with 3'-alkyne terminated oligonucleotide coding units and templated or non-templated Click chemistry (El-Sagheer, et al. (2011), "Biocompatible Artificial DNA Linker That is Read Through by DNA Polymerases and is Functional in Escherichia coli", PNAS 108(28):11338-11343) to assemble DNA nucleic acid barcodes. In some instances, a barcode initiator moiety may comprise a modified nucleotide that is functionalized with, *e.g.,* a primary amine, a carboxyl group, a thiol group, *etc.,* which serves an initiation site for subsequent covalent conjugation of amino acid or peptide coding units to assemble DNA nucleic acid barcodes. In some instances, the second sequence region of the primer may comprise a common linker sequence that allows for annealing of the primer to a "splint" molecule *(e.g.,* a short oligonucleotide sequence) that is also complementary to a common liner sequence attached to an oligonucleotide coding unit. Annealing of the primer, splint, and coding unit oligo then positions the primer and coding unit oligo such that they may be joined by ligation, and repeated cycles of annealing, ligation, and denaturation may be used to assemble DNA nucleic acid barcodes. The use of splints (or "templates") to assemble cell origination oligonucleotide barcode sequences has been described in a co-pending PCT application WO 2012/106385.

*Primer binding sites:* In some instances, the amplification and/or sequencing primer binding site regions may range from about 18 to about 30 nucleotides in length, and preferably between about 20 and 25 nucleotides in length, with a melting temperature (Tm) of between 65°C and 75°C. Use of sequences having more GC content or longer length will shift the Tm to higher temperatures. Typically, the GC content of the primer sequence will be between 40% and 60%, with the 3'-end of the primer ending in C or G to promote binding. In some instances, the sequencing primer binding site may comprise a sequence that is complementary to an Illumina sequencing primer.

*Spacer or linker sequences:* In some instances, the barcode initiation primers used in the disclosed DNA barcoding methods may further comprise a spacer sequence or linker sequence used to separate the first and second sequence regions, or for attachment of a detectable label. In general, the length of the spacer and/or linker sequences may range from about 2 nucleotides to about 20 nucleotides. In some instances, the length of the spacer and/or linker sequences may be at least 2 nucleotides, at least 4 nucleotides, at least 6 nucleotides, at least 8 nucleotides, at least 10 nucleotides, at least 12 nucleotides, at least 14 nucleotides, at least 16 nucleotides, at least 18 nucleotides, or at least 20 nucleotides. In some instances, the length of the spacer and/or linker sequences may be at most 20 nucleotides, at most 18 nucleotides, at most 16 nucleotides, at most 14 nucleotides, at most 12 nucleotides, at most 10 nucleotides, at most 8 nucleotides, at most 6 nucleotides, at most 4 nucleotides, or at most 2 nucleotides. Any of the lower and upper values described in this paragraph may be combined to form a range included within the disclosure, for example, the length of the spacer and/or linker sequences may range from about 4 nucleotides to about 16 nucleotides. Those of skill in the art will recognize that the length of the spacer and/or linker sequences may have any value within this range, e.g., about 7 nucleotides.

*Overall length of barcode initiation primers:* The overall length of the random, semi-random, or targeted barcode initiation primers comprises the length of the region that is complementary to the target *(e.g.,* the template DNA), and the length of additional sequence regions used to incorporate common linker sequences, random sequence regions, amplification or sequencing primer binding sequences, *etc.* In some instances, the overall length of the random, semi-random, or targeted barcode initiation primer molecules used in the disclosed DNA barcoding methods may range from about 5 nucleotides to about 80 nucleotides. In some instances, the length of the barcode initiation primer may be at least 5 nucleotides, at least 10 nucleotides, at least 15 nucleotides, at least 20 nucleotides, at least 25 nucleotides, at least 35 nucleotides, at least 40 nucleotides, at least 45 nucleotides, at least 50 nucleotides, at least 55 nucleotides, at least 60 nucleotides, at least 65 nucleotides, at least 70 nucleotides, at least 75 nucleotides, or at least 80 nucleotides. In some instances, the length of the barcode initiation primer may be at most 80 nucleotides, at most 75 nucleotides, at most 70 nucleotides, at most 65 nucleotides, at most 60 nucleotides, at most 55 nucleotides, at most 50 nucleotides, at most 45 nucleotides, at most 40 nucleotides, at most 35 nucleotides, at most 30 nucleotides, at most 25 nucleotides, at most 20 nucleotides, at most 15 nucleotides, at most 10 nucleotides, or at most 5 nucleotides. Any of the lower and upper values described in this paragraph may be combined to form a range included within the disclosure, for example, the length of the barcode initiation primer may range from about 10 nucleotides to about 40 nucleotides. Those of skill in the art will recognize that the length of the barcode initiation primer may have any value within this range, *e.g.,* about 28 nucleotides.

*Synthesis of complementary DNA strands:* As noted above, complementary DNA strands that comprise complementary sequences to all or a portion of the template DNA nucleic acids may be synthesized by performing primer extension reactions, *i.e.,* by incorporation of nucleotides at the 3'-OH end of barcode initiation primer molecules annealed to the template DNA. In some instances, a plurality of barcode initiation primer molecules may be hybridized to each template DNA nucleic acid under suitable hybridization conditions, and a plurality (or "set") of barcoded, complementary DNA sequences may be prepared by performing a polymerase extension reaction for each of the annealed barcode initiation primers to synthesize DNA that is complementary to all or a portion of the template DNA nucleic acid. Examples of suitable DNA polymerases for use in primer extension include, but are not limited to, the Klenow fragment of DNA polymerase 1 (which lacks the 5'→3' exonuclease activity of DNA polymerase 1), Taq polymerase (commonly used for polymerase chain reaction (PCR)-based methods because of its thermal stability), Pfu DNA polymerase (also used for PCR due to its superior thermostability and proofreading properties compared to Taq polymerase), and the like, or combinations thereof. The primer extension reaction is performed at a temperature which maintains stable primer binding and optimizes the activity of the polymerase used, for example, Taq polymerase has optimal activity at 75-80 °C, and reaction temperature of about 72 °C is used with this enzyme. The DNA polymerase synthesizes a new DNA strand complementary to the template DNA nucleic acid by adding deoxynucleotide triphosphates (dNTPs) that are complementary to the template in the 5' to 3' direction, condensing the 5'-phosphate group of a dNTP with the 3'-hydroxyl group at the end of the growing complementary DNA strand. Extension reaction times vary depending on the DNA polymerase used and on the length of the DNA nucleic acids to be copied (DNA polymerase incorporates approximately a thousand bases per minute under optimal conditions). In addition to the template DNAs nucleic acid, primers, a DNA polymerase, and dNTPs, the extension reaction mixture will typically include an appropriate buffer, bivalent cations, e.g., magnesium, and monovalent cations, e.g., potassium ions. In some instances, extension reactions may be performed in a small reaction volume of approximately 10-200 µl in reaction tubes having volumes of approximately 0.2-0.5 ml.

In some instances, it may be desirable to block further extension of the complementary DNA strand at random positions (e.g., in order to avoid displacement by the polymerase of additional complementary strands being synthesized on the same template DNA fragment) by introducing a dideoxy nucleotide triphosphate (ddNTP) into the reaction mixture at appropriate concentrations and/or times. For example, introduction of a ddCTP will block further extension of the complementary DNA strand at the position of the next G residue in the template DNA due to the absence of a free 3'-OH group, thereby terminating the growing complementary DNA strand.

In some instances, e.g., when the target is RNA, a reverse transcriptase (RT) may be used to perform primer extension reactions for barcode initiation primers that are annealed to the RNA template. Any of several suitable reverse transcriptases known to those of skill in the art may be used including, but not limited to, Avian Myeloblastosis Virus (AMV) Reverse Transcriptase and Moloney Murine Leukemia Virus (M-MuLV, MMLV) Reverse Transcriptase (New England Biolabs, Ipswich, MA). M-MuLV Reverse Transcriptase (also from New England Biolabs, Ipswich, MA) lacks a 3'→5' exonuclease activity. ProtoScript^{®} II Reverse Transcriptase (New England Biolabs, Ipswich, MA) is a recombinant M-MuLV reverse transcriptase with reduced RNase H activity and increased thermostability, and can be used to synthesize first strand cDNA at higher temperatures than the wild-type enzyme. The use of engineered RTs improves the efficiency of full- length product formation, thereby ensuring that copying of the 5'-end of mRNA transcripts is complete, and enables synthesis and characterization of accurate cDNA copies of RNA template sequences. The use of the more thermostable RTs, where reverse transcription reactions may be performed at higher temperatures, facilitates transcription of RNA that contains high amounts of secondary structure.

*Split-pool synthesis of DNA barcodes:* As used herein, the phrase "split-pool synthesis" refers to one non-limiting example of a combinatorial synthesis process in which a reaction mixture is divided into several different aliquots prior to performing a coupling reaction, and wherein each aliquot receives a different chemical monomer *(i.e.,* an assayable polymer subunit (APS) or coding subunit) to be coupled. Following the coupling reaction, the aliquots are combined (pooled), mixed, and divided (split) into a new set of aliquots prior to performing the next round of coupling. In general, the approach may be used for a variety of coupling reactions and conjugation chemistries including, but not limited to, amino acid (or short peptide) coupling reactions to produce longer peptides of fully or partially random amino acid sequences, the coupling of deoxyribonucleotides (or short DNA oligonucleotides) to produce longer DNA oligonucleotides of fully or partially random base sequences, or the coupling of ribonucleotides (or short RNA oligonucleotides) to produce longer RNA oligonucleotides of fully or partially random base sequences. Any of a variety of chemical monomers, *e.g.,* amino acids, small molecules, short peptides, short oligonucleotides, etc., may thus be used as building blocks for assembly of unique barcodes. In a preferred embodiment, the APS used in successive rounds of split-pool synthesis comprise uniquely designed nucleic acid sequences. An advantage of the template DNA nucleic acid barcode assembly and synthesis methods disclosed herein is that there is no requirement that individual template DNA nucleic acids be partitioned into individual reaction compartments prior to performing barcode assembly and synthesis reactions.

The upper part of FIG. 2 illustrates the first round of a split-pool synthetic route to creating unique template DNA nucleic acid barcodes. Following the synthesis of a plurality of complementary DNA strands using a plurality of barcode initiation primer molecules annealed to each template DNA nucleic acid, the sample is divided into a series of aliquots and a first assayable polymer subunit (APS), *e.g.,* a short oligonucleotide coding unit, is coupled to the barcode synthesis initiation site on each of the annealed complementary DNA / template DNA complexes within the sample aliquot, wherein each sample aliquot is treated with a different APS.

The lower part of FIG. 2 illustrates subsequent rounds of a split-pool synthetic route to creating unique template DNA nucleic acid barcodes. Following the first APS (or coding unit) coupling round, the sample aliquots are pooled, mixed, and dispensed into a new series of aliquots. A second coding unit is then coupled to the first coding unit on each of the annealed complementary DNA / template DNA complexes, wherein again each sample aliquot is treated with a different APS (or coding unit). Performing several successive rounds of split-pool synthesis thus results in the creation of a set of substantially unique template DNA nucleic acid barcodes that encode the identities of individual template DNA nucleic acids from the original sample.

The diversity of the template DNA barcode library *(i.e.,* the number of unique template DNA nucleic acid barcodes that are theoretically possible) that can be achieved by means of performing stepwise split-pool assembly and synthesis is dependent on the number of unique APS (or coding units) available for use in each round, and the total number of rounds used to assemble the template DNA nucleic acid barcode. For example, for a template DNA barcode created using four rounds of assembly/synthesis *(i.e.,* for a template DNA barcode having four APS positions) and 100 unique APS, the total number of unique template DNA barcode sequences that are possible is 100⁴ = 10⁸ or 100,000,000. Alternatively, for a template DNA barcode created using six rounds of assembly/synthesis *(i.e.,* for a template DNA barcode having six APS positions) and 100 unique APS, the total number of unique template DNA barcode sequences that are possible is 100⁶ = 10¹² or 1,000,000,000,000. In general, it is desirable to design the template DNA barcode library such that the total number of unique sequences available is significantly larger than the number of individual template DNA nucleic acids to be labeled, thereby ensuring that the probability that any two template DNA nucleic acids are labeled with the same template DNA barcode is extremely low. In some cases, it may be possible to use fewer barcodes given the non-redundancy of the genome and usage of known sequence assemblies.

In general, the number of rounds of split-pool synthesis used to create unique template DNA nucleic acid barcodes may range from about 2 to about 40 rounds. In some instances, at least 2, at least 4, at least 6, at least 8, at least 10, at least 12, at least 14, at least 16, at least 18, at least 20, at least 25, at least 30, at least 35, or at least 40 rounds of split-pool synthesis may be performed. In some instances, at most 40, at most 35, at most 30, at most 25, at most 20, at most 18, at most 16, at most 14, at most 12, at most 10, at most 8, at most 6, at most 4, or at most 2 rounds of split-pool synthesis may be performed. Any of the lower and upper values described in this paragraph may be combined to form a range included within the disclosure, for example, the number of rounds of split-pool synthesis performed may range from about 4 to about 12. Those of skill in the art will recognize that the number of rounds of split-pool synthesis performed may have any value within this range, e.g., about 7 rounds.

In general, the pool of unique APS used for coupling in each round of split-pool synthesis may comprise from about 2 to about 200 unique APS. In some instances, the number of unique APS in the pool may be at least 2, at least 4, at least 6, at least 8, at least 10, at least 20, at least 30, at least 40, at least 50, at least 100, at least 150, or at least 200. In some instances, the number of unique APS in the pool may be at most 200, at most 150, at most 100, at most 100, at most 50, at most 40, at most 30, at most 20, at most 10, at most 8, at most 6, at most 4, or at most 2. Any of the lower and upper values described in this paragraph may be combined to form a range included within the disclosure, for example, the number of unique APS in the pool may range from about 4 nucleotides to about 20. Those of skill in the art will recognize that the number of unique APS in the pool may have any value within this range, e.g., about 12 unique APS.

As indicated above, in some instances, the APS (or coding units used to assemble the barcode) of the template DNA nucleic acid barcodes are covalently or non-covalently coupled in a serial, linear fashion to one end of the growing barcode molecule. In some instances, the APS (or coding units) are stitched together and/or annealed to a common linker (CL) sequence using an annealing primer *(i.e.,* a template molecule or "splint") (FIG. 3). In some instances, the annealing primer may comprise a sequence region *(i.e.,* a first complementary region) that is complementary to an APS added during the previous round of stepwise synthesis. In some instances, the annealing primer may also comprise a second sequence region *(i.e.,* a second complementary region) that is complementary to the APS being added during a current round. Thus, the annealing primer can hybridize to two or more oligonucleotide subunits of successive rounds, which in some instances may be followed by a ligation reaction, thereby stitching them together. In some embodiments, the first complementary regions of the annealing primers of each round are different from the first complementary regions of annealing primers of other rounds. In some embodiments, the second complementary regions of the annealing primers of each round are different from the second complementary regions of annealing primers of other rounds. In some embodiments, the first or second complementary regions of the annealing primers of different rounds are shared between rounds. In some embodiments, a template or "splint" *(i.e.,* an extended CL molecule) is used for assembly of APS, wherein the splint includes multiple sets of annealing regions *(e.g.* common linker sequences) designed to permit the stepwise hybridization and ligation of individual APS (or coding subunits) to create the completed template DNA barcode.

In some instances, a CL or "splint" oligonucleotide comprises one or more pairs of loop annealing regions. In these instances, the APS may be designed to hybridize to the CL or splint to create loop geometries, *i.e.,* by hybridizing to the CL loop annealing regions at each end of the APS (FIG. 5). In some instances, the loop annealing regions may be designed to be specific to the round of split-pool synthesis such that successive rounds of addition and hybridization populate the APS positions along the splint. The APS can then be linked together using any of the methods known in the art, for example, by ligation. In some instances, the APS may be designed to ensure that they do not hybridize efficiently to the splint at the loop annealing regions specific to other synthesis rounds. Consequently, if an APS from a particular round is missing for some reason, APS that are added in subsequent rounds are less likely to be ligated properly, thus reducing the likelihood of downstream analysis errors. Alternatively, template DNA barcodes may occasionally be synthesized even with a missing APS, the location of which is flanked by a pair of loop annealing regions. The resulting template DNA barcode can then be analyzed accordingly, and can either be discarded or the retrieved information can be alternatively processed.

*Random or semi-random tag sequences:* In some instances, the plurality of APS may further comprise a random or semi-random tag sequence in addition to the coding sequence, where the random or semi-random tag sequences for each APS associated with a given template DNA nucleic acid barcode function as a unique molecular counter sequence to identify an individual strand of barcoded, complementary DNA that is derived from a given template DNA nucleic acid. In some instances, the number of different molecular counter sequences associated with each template DNA nucleic acid barcode sequence may be used to enumerate or confirm the initial number of complementary DNA copies that were derived from a given template DNA nucleic acid *(i.e.,* the number of complementary DNA copies that existed prior to performing subsequent amplification reactions), or to detect repeat sequences within a given template DNA nucleic acid. In some instances, such molecular counter sequences may be used to determine the probability that a template nucleic acid fragment sequence variant was derived from a polymerase error, *e.g.,* strand switching, during an amplification reaction. In some instances, a random or semi-random tag sequence may be incorporated into the barcode initiation primer molecules.

In some instances, the random or semi-random tag sequence may range from about 2 to about 8 nucleotides in length. In some instances, the random or semi-random tag sequence may be at least 2, at least 4, at least 6, or at least 8 nucleotides in length. In some instances, the random or semi- random tag sequence may be at most 8, at most 6, at most 4, or at most 2 nucleotides in length. Any of the lower and upper values described in this paragraph may be combined to form a range included within the disclosure, for example, the random or semi-random tag sequence may range from about 4 nucleotides to about 6 nucleotides in length. Those of skill in the art will recognize that length of the random or semi-random tag sequence may have any value within this range, e.g., about 7 nucleotides.

*Error checking:* In some instances, the plurality of APS may comprise a set of uniquely designed nucleic acid sequences comprising one or more sub-code (SC) regions (FIG. 4), wherein the sub-code sequence is unique for each individual APS molecule in the plurality of APS. In some instances, the SC regions or sequences are about 3, 4, 5, 6, 7, 8, 9, 10, or more than 10 nucleotides in length. In some instances, the sub-codes comprise a unique set of nucleic acid sequences of defined length, *e.g.,* 7 nucleotides, which are designed to provide error correction capability. In some instances, the set of sub-codes comprise 7 nucleotide sequences designed such that any pairwise combination of sequences in the set exhibits a defined "genetic distance", or number of mismatched bases, *e.g.,* a distance of 3. In this case, review of the sub-codes in the set of template DNA fragment barcodes identified in the final step of an assay allows one to detect hybridization or amplification errors prior to performing the final analysis of the assay data. In some instances, a random sequence region might be included adjacent to the APS code, but not as part of the annealing region.

*Amplification reactions:* In some instances of the disclosed DNA barcoding methods, one or more nucleic acid amplification reactions may be performed to create multiple copies of the template DNA nucleic acids or barcoded complementary DNA strands thereof. In some instances, amplification may be performed in a multiplexed manner, wherein multiple template DNA nucleic acid sequences or barcoded complementary DNA strands are amplified simultaneously. In some instances, the amplification reaction may be used to add sequencing adaptors to the nucleic acid molecules. The amplification reactions may comprise amplifying at least a portion of a barcode, if present. The amplification reactions may comprise amplifying at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, or 100% of the plurality of the template DNA nucleic acids or barcoded complementary DNA strands thereof.

In some instances, amplification of specific oligonucleotide sequences may be performed using a polymerase chain reaction (PCR). As used herein, PCR may refer to a reaction for the *in vitro* amplification of specific DNA sequences by simultaneous primer extension of complementary strands of DNA. As used herein, PCR may encompass derivative forms of the reaction, including but not limited to, reverse-transcriptase PCR (RT-PCR), real-time PCR, nested PCR, quantitative PCR, multiplexed PCR, digital PCR, and assembly PCR.

In some instances, amplification of specific oligonucleotide sequences may comprise non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR based amplification methods include performing multiple cycles of DNA-dependent RNA polymerase-driven RNA transcription amplification or RNA-directed DNA synthesis and transcription to amplify DNA or RNA targets, a ligase chain reaction (LCR), a Q replicase (Q ) method, use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and ramification extension amplification (RAM).

Conducting the one or more amplification reactions may comprise the use of one or more amplification primers. As indicated above, the one or more amplification primers may range in length from about 10 nucleotides to about 40 nucleotides. In some instances, the length of the amplification primer may be at least 10 nucleotides, at least 15 nucleotides, at least 20 nucleotides, at least 25 nucleotides, at least 30 nucleotides, at least 35 nucleotides, or at least 40 nucleotides. In some instance, the length of the amplification primer may be at most 40 nucleotides, at most 35 nucleotides, at most 30 nucleotides, at most 25 nucleotides, at most 20 nucleotides, at most 15 nucleotides, or at most 10 nucleotides. Any of the lower and upper values described in this paragraph may be combined to form a range included within the disclosure, for example, the length of the amplification primer may range from about 15 nucleotides to about 25 nucleotides. Those of skill in the art will recognize that the length of the amplification primer may have any value within this range, e.g., about 22 nucleotides.

The one or more amplification primers may anneal to at least a portion of the plurality of barcoded nucleic acid sequences. The one or more primers may anneal at or near the 3'-end of the plurality of barcoded nucleic acid sequences. The one or more primers may anneal nearer the 5'-end of the plurality of barcoded nucleic acid sequences. The one or more primers may anneal to an internal region of the plurality of barcoded nucleic acid sequences. The one or more amplification primers may comprise a fixed panel of primers. The one or more amplification primers may comprise one or more custom primers. The one or more custom primers may be designed to anneal to one or more target nucleic acid sequences, e.g., one or more gene sequences. The one or more amplification primers may comprise one or more control primers. The one or more control primers may comprise at least one or more housekeeping gene primers. The one or more amplification primers may comprise a universal primer. The universal primer may anneal to a universal primer binding site. The one or more amplification primers may anneal to a first template DNA nucleic acid barcode, a second template DNA nucleic acid barcode, another template DNA nucleic acid barcode, an amplification or universal primer binding site that has been incorporated into all template DNA nucleid acid barcodes, a first template DNA nucleic acid sequence, a second template DNA nucleic acid sequence, another template DNA nucleic acid sequence, *etc.,* or a combination thereof. The one or more amplification primers may comprise a universal primer and one or more custom primers.

In some aspects, determining the number of different barcoded template DNA nucleic acids may comprise determining the sequence of the barcoded template DNA nucleic acids or any product thereof (e.g., barcoded-amplicons). Determining the sequence of the barcoded template DNA nucleic acids, or any product thereof (e.g., barcoded amplicons), may comprise conducting a sequencing reaction to determine the sequence of at least a portion of the barcode, at least a portion of the barcoded template DNA nucleic acid sequence, a complement thereof, a reverse complement thereof, or any combination thereof.

*Sequencing:* In some aspects of the disclosed methods, determining the spatial relationship between two or more specified template DNA sequences, *e.g.,* their proximity within a given segment of a genome as indicated by co-location within a single template DNA nucleic acid, may comprise identifying those template sequences that are associated with the same complementary DNA strand barcode. In some aspects of the disclosed methods, determining the copy number for a specified template DNA sequence, *e.g.,* a specific gene sequence, in a sample may comprise determining the number of unique complementary DNA barcode sequences associated with the specified template DNA sequence. Determining the sequence of the barcoded complementary DNA strands, or any product thereof *(e. g.,* barcoded amplicons), may comprise conducting a sequencing reaction to determine the sequence of at least a portion of the barcode, at least a portion of the barcoded complementary DNA strand, a complement thereof, a reverse complement thereof, or any combination thereof.

Determination of the sequence of a nucleic acid *(e.g.,* a barcoded complementary DNA strand, or barcoded amplicons thereof) may be performed using any of a variety of sequencing methods including, but not limited to, sequencing by synthesis (SBS) *(e.g.,* Sanger sequencing or pyrosequencing), sequencing by hybridization (SBH), sequencing by ligation (SBL), cyclic array-based sequencing, polymerized colony (POLONY) sequencing, and the like.

In some instances, determining the sequence of the barcoded complementary DNA strands, or any product thereof, may comprise the use of paired-end sequencing, shotgun sequencing, high-throughput sequencing, nanopore-based sequencing, dye-terminator sequencing, multiple-primer DNA sequencing, primer walking, Sanger dideoxy sequencing, Maxim-Gilbert sequencing, pyrosequencing, true single molecule sequencing, or any combination thereof. Alternatively, the sequence of the barcoded complementary DNA strands, or any product thereof, may be determined in some instances using a microarray chip, by electron microscopy or using a chemical-sensitive field effect transistor (chemFET) array.

High-throughput sequencing methods, such as cyclic array sequencing using platforms such as the Roche 454, Illumina Solexa, ABI-SOLiD, Ion Torrent, Complete Genomics, Pacific Bioscience, Helices, or the Polonator platform, may also be utilized. In some instances, sequencing may comprise Illumina MiSeq sequencing.

In some instances, sequencing the barcoded complementary DNA strands generated by the disclosed DNA barcoding methods may comprise sequencing complementary DNA sequences representing from about 0.01% of the genes of an organism's genome to about 100% of the genes of an organism's genome. In some instances, the complementary DNA that is sequenced may comprise at least 0.01% of an organism's genome, at least 0.1% of an organism's genome, at least 1% of an organism's genome, at least 5% of an organism's genome, at least 10% of an organism's genome, at least 20% of an organism's genome, at least 30% of an organism' s genome, at least 40% of an organism's genome, at least 50% of an organism's genome, at least 60% of an organism' s genome, at least 70% of an organism' s genome, at least 80% of an organism' s genome , at least 90% of an organism's genome, or at least 95% of an organism's genome. In some instances, the complementary DNA that is sequenced may comprise at most 95% of an organism ' s genome , at most 90% of an organism' s genome, at most 80% of an organism' s genome , at most 70% of an organism's genome, at most 60% of an organism's genome, at most 50% of an organism's genome, at most 40% of an organism's genome, at most 30% of an organism's genome, at most 20% of an organism's genome, at most 10% of an organism's genome, at most 5% of an organism's genome, at most 1% of an organism's genome, at most 0.1% of an organism' s genome, or at most 0.01% of an organism ' s genome. Any of the lower and upper values described in this paragraph may be combined to form a range included within the disclosure, for example, the complementary DNA that is sequenced may comprise from about 5% to about 40% of an organism's genome. Those of skill in the art will recognize that the complementary DNA that is sequenced may comprise any value within this range, e.g., about 63% of an organism's genome.

In some instances, sequencing may comprise sequencing at least about 10, 20, 30, 40, 50, 60, 70, 80, 90, 100, 200, 300, 400, or more nucleotides or base pairs of the barcoded nucleic acid sequences. In some instances, sequencing may comprise sequencing at least about 500, 600, 700, 800, 900, 1,000 or more nucleotides or base pairs of the barcoded nucleic acid sequences. In other instances, sequencing comprises sequencing at least about 1,500, 2,000, 3,000, 4,000, 5,000, 6,000, 7,000, 8,000, 9,000, or 10,000 or more nucleotides or base pairs of the barcoded nucleic acid sequences.

In some instances, sequencing may comprise at least about 100, 1,000, 10⁴, 10⁵, 10⁶, 10⁷, 10⁸, 10⁹ or 10¹⁰ or more sequencing reads per run. In some instances, sequencing may comprise less than or equal to about 1,000,000,000 sequencing reads per run. In some instances, sequencing may comprise less than or equal to about 100,000,000 reads per run. In some instances, sequencing may comprise less than or equal to about 10,000,000 reads per run. In some instances, sequencing may comprise less than or equal to about 1,000,000 reads per run. In some instances, sequencing may comprise less than or equal to about 100,000 reads per run. In some instances, sequencing may comprise less than or equal to about 10,000 reads per run.

*Sequence Assembly:* Assembly of longer DNA or other oligonucleotide sequences, e.g., genome fragments or whole genomes, from relatively short sequence reads is performed by identifying the overlapping sequences from multiple short sequence reads to assemble longer, contiguous sections of sequence. In some instances, comparison to a known reference sequence or consensus sequence from the same or a similar organism may be used to identify gaps or errors in the assembled sequence. Any of a variety of bioinformatics software programs known to those of skill in the art may be used to assemble longer sequences from relatively short sequence reads.

Examples include, but are not limited to, DBG2OLC, SPAdes, SparseAssembler, Fermi, and SGA. In the present disclosure, the barcode sequence associated with each short template DNA sequence read facilitates identification of those sequences that arose from the same template DNA nucleic acid, and therefor facilitates the template sequence assembly process for mapping whole genomes or portions thereof.

*Phasing and haplotyping:* Whole-genome sequencing typically generates a single consensus sequence without distinguishing between the sequence variants, *e.g.,* single nucleotide polymorphisms (SNPs), insertions, deletions, and/or mutations, found on different homologous chromosomes, *e.g.,* those derived from the maternal and paternal lines of inheritance. Phased sequencing addresses this limitation by determining which genetic variants appear on the same or different chromosomes and are thus inherited together or separately. Information about patterns of genetic variation, such as haplotype *(i.e.,* a group of sequence variants that are inherited as a contiguous block), is important in understanding genetically-inherited traits and genetic disease, as is information about the copy number of specific genes. Phased sequencing provides an approach for determining haplotype as well as identifying the presence of *de novo* mutations, and thus has applications in studies of population genetics and genetic disease.

As noted above, the term "haplotype" refers to sets of DNA sequence variants (alleles) that are inherited together in contiguous blocks. In general, the human genome contains two copies of each gene - a maternal copy and a paternal copy. For a pair of genes each having two possible alleles, for example gene alleles "A" and "a", and gene alleles "B" and "b", the genome of a given individual will include one of two haplotypes, "AB/ab", where the A and B alleles reside on the same chromosome (the "cis" configuration), or "Ab/aB", where the A and B alleles reside on different chromosomes (the "trans" configuration). Phased sequencing methods or assays can be used to determine whether a specified set of gene alleles reside on the same or different chromosomes. In some cases, several linked alleles that define a haplotype may correlate with, or be associated with, a particular disease phenotype; in such cases, a haplotype, rather than any one particular genetic variant, may be the most determinative factor as to whether a patient will display the disease.

Many genetic diseases, and in particular, cancer, are associated with chromosomal rearrangements such as deletions, insertions, duplications, translocations, and inversions (Lee, et al. (2012), "Targeted Chromosomal Duplications and Inversions in the Human Genome Using Zinc Finger Nucleases", Genome Research 22:539-548). These genome structural variations (SVs) are observed in healthy individuals as well as disease patients, and thus contribute to both genetic diversity and the onset of disease.

Gene copy number also plays a role in some disease phenotypes. Most genes are normally present in two copies, however, "amplified" genes are genes that are present in more than two functional copies and therefore lead to the production of abnormal levels of mRNA and protein expression, potentially leading to a cancerous state. Cancer and other genetic disorders are often correlated with abnormal *(i. e.,* increased or decreased) numbers of chromosome ("aneuploidy"). Cytogenetic techniques such as fluorescence *in situ* hybridization or comparative genomic hybridization are typically used to detect the presence of abnormal gene or chromosome copy numbers.

*Use of barcoded DNA fragments for phased sequencing:* The methods disclosed herein provide a means for producing a plurality of barcoded DNA molecules, wherein each barcoded DNA molecule comprises at least a first sequence region, a second sequence region, and a third sequence region. The first sequence region is the barcode sequence assembled using a split-pool synthesis approach, as described above, wherein the barcode sequence provides a unique identifier for the original template DNA nucleic acid from which the DNA molecule originated. The second sequence region is the complement of a random or semi-random primer sequence, or a target-specific primer sequence, that was used to prime the original template DNA nucleic acid. The third sequence region is a stretch of DNA that is complementary to all or a portion of the original template DNA nucleic acid. The barcode sequence may thus be used to identify the plurality of complementary DNA molecules that arose from a single template DNA nucleic acid. Subsequent alignment and assembly of the associated complementary DNA sequences thus allows one to determine the sequence for stretches of the original template DNA that are substantially longer than typical sequencing read lengths. In some instances, the disclosed DNA barcoding methods for long-range sequencing may be used to identify those DNA sequences (or complements thereof) that are associated with the same DNA strand and/or the same chromosome without the need for partitioning individual DNA strands or chromosomes into separate compartments prior to performing amplification and sequencing reactions. In some instances, the barcode information may be used in conjunction with one or more known marker gene sequences to identify those DNA sequences (or complements thereof) that are associated with the one or more known marker gene sequences on the same DNA strand and/or the same chromosome, again without the need for partitioning individual DNA strands or chromosomes into separate compartments prior to performing amplification and sequencing reactions. In some instances, conclusions regarding the phasing of particular genetic variants may then be drawn. Such information may be useful for identifying haplotypes, *i.e.,* a specified set of genetic variants that reside on the same nucleic acid strand or on different nucleic acid strands. In some instances, the identification of a specific set of genetic structural variants, *e.g.,* deletions, insertions, duplications, translocations, and inversions, which reside on the same nucleic acid strand or on different nucleic acid strands, may be used for the diagnosis of genetic disease, *e.g.,* cancer. In some instances, determination of the number of unique barcode sequences associated with one or more specified known gene sequences may be used to identify copy number variations for those genes.

The disclosed methods provide significant advantages over current nucleic acid sequencing technologies and their associated sample preparation methods. In addition to facilitating the assembly of longer DNA sequences from short sequencing reads, the barcoding methods of the present disclosure may potentially be applied to determination of haplotype phasing and copy number variation, as noted above. Haplotype phasing and copy number variation data are generally not available by sequencing genomic DNA because biological samples *(e.g.,* blood, cells, or tissue samples) are typically processed in bulk to extract the genetic material from a large number of cells, and convert it into sequencing libraries that are configured specifically for the input requirements of a given sequencing technology. As a result of this bulk sampling and processing approach, sequencing typically provides non-phased consensus sequence data for which it is not possible to determine whether specific genetic variants are present on the same or different chromosomes. Recently, several publications have disclosed methods for creating barcoded cDNA and performing phasing and haplotyping (see, for example, US 8,268,564; WO2014/124338; WO2014/210353; WO2015/200869; and WO2015/200893). In general, the latter methods rely on the use of pre-synthesized barcodes and the partitioning of the pre-synthesized barcodes and cells, or nucleic acids isolated therefrom, into individual reaction compartments, e.g., the droplets formed by a water-in-oil emulsion.

A number of studies have demonstrated that relationships between human genomic DNA sequence and phenotype, including disease, can be more fully understood when phase information is available (Tewhey, et al. (2011), "The Importance of Phase Information for Human Genomics", Nat. Rev. Genet. 12(3):215-223). Applications of phased sequencing and haplotyping include determining genotype and detecting genotype error, understanding the interplay of genetic variation (e.g., structural variations such as deletions, insertions, duplications, translocations, and inversions) and disease, inferring the presence of previously uncharacterized genetic variation, inferring human demographic history, inferring points of recombination, detecting recurrent mutation and signatures of selection , and modeling cis-regulation of gene expression (Brown and Brown (2011), "Haplotype Phasing: Existing Methods and New Developments", Nat. Rev. Genet. 12(10):703-714).

### Example - Long-Range Sequencing Using Barcoded Complementary DNA

The DNA barcoding method of the present disclosure is illustrated in FIGS. 1 A-C. A fragmented DNA sample, *e.g.,* fragmented genomic, exomic, or other type of DNA, is melted or denatured *(i.e.,* to create single-stranded DNA) and end-labeled with biotin at the 3'-end for later purification (FIG. 1 A). This step may be completed in approximately 30 minutes.

Following the biotin-labeling step, the template DNA nucleic acid fragments are primed using a plurality of barcode initiation primers, *e.g.,* primer molecules comprising a random or semi-random template annealing region and a barcode initiation site or moiety (FIG. 1 B). Following the annealing of one or more barcode initiation primers to each template DNA nucleic acid fragment, a polymerase extension reaction is performed to create a "set" of complementary DNA strands annealed to the template DNA. The annealing and primer extension reactions may all be performed in a single tube or reaction vessel. In some instances, a dideoxy nucleotide, *e.g.,* ddCTP, may be introduced to the reaction mixture to create random stops that prevent the polymerase from displacing other complementary DNA strands attached to the same template DNA nucleic acid. This step may be completed in approximately 30minutes.

Following the primer extension step, each set of complementary DNA strands is barcoded by performing a split-pool synthesis or assembly reaction using a set of APS (or coding units) as described above (FIGS. 1 C and 2). The number of rounds of split-pool synthesis and the number of unique APS available for coupling at each round of synthesis are selected to ensure that the probability of any two template DNA nucleic acids (or sets of complementary DNA strands annealed to the two template DNA nucleic acids) having the same barcode is extremely small. Thus each template DNA nucleic acid (or set of complementary DNA strands annealed to each template DNA nucleic acid) has a substantially unique barcode that may subsequently be used to identify the set of complementary DNA sequences that were synthesized from a single template DNA nucleic acid fragment. The split-pool synthesis of template DNA fragment barcodes may be performed, for example, in a simple microfluidic device or in a 96 well plate using a multichannel pippetor. There are no separation or centrifugation steps required (beyond pooling and re-aliquoting of the sample), and no need to partition individual template DNA nucleic acids into separate reaction vessels or compartments. This step may be performed in approximately 1 hour.

Following the synthesis of the barcodes, the sets of barcoded complementary DNA strands annealed to the template DNA nucleic acids may be affinity purified using any of a variety of biotin/avidin purification schemes, the barcoded complementary DNA strands are denatured and amplified, *e.g.,* using PCR and semi-random priming of the complementary strands, sequenced, and long-range sequences may be assembled by using the barcodes to identify sets of complementary sequences that were synthesized from a single template DNA nucleic acid.

## Claims

1. A method for facilitating nucleic acid sequence assembly, the method comprising:
a) providing a nucleic acid sample comprising single-stranded template nucleic acids;
b) priming each template nucleic acid with one or more barcode initiation primers to create annealed nucleic acid assemblies, wherein each barcode initiation primer comprises a 3' - template hybridization portion, and a 5'- barcode initiation portion;
c) performing a polymerase extension to create template complementary strands which remain annealed to the template nucleic acids;
d) performing two or more rounds of split-pool synthesis coupling oligonucleotide coding units "assayable polymer subunits" (APS) to assemble an oligonucleotide barcode sequence on each of the template complementary strands comprising the barcode region, the primer region and the target-specific region complementary to the target nucleic acid;
e) sequencing the barcoded complementary strands to obtain template DNA sequence reads; and
f) , identifying barcode sequence associated with each template DNA sequence read to identify those sequences that arose from the same template nucleic acid and assembling the template nucleic acid sequence from the template-complementary regions of the barcoded complementary strands.

2. The method of claim 1, further comprising end-labeling the single-stranded template nucleic acids at the 3'-end with an affinity tag prior to step (b).

3. The method of claim 1 or claim 2, wherein the affinity tag is biotin.

4. The method of any one of the preceding claims, wherein the 3'-barcode initiation portion comprises a random sequence.

5. The method of any one of the preceding claims, wherein the polymerase extension in step c) comprises a dideoxy-nucleotide.

6. The method of any one of the preceding claims, wherein the APS include error checking sub-codes (SC) comprising a defined sequence of about 7 nucleotides that differs from other SC in the sample by at least 3 nucleotides.

7. The method of any one of the preceding claims, wherein each APS comprises a random sequence.

8. The method of any of the preceding claims, further comprising a step of counting the barcoded complementary strands that arose from the same template nucleic acid thereby detecting the initial number of copies of the template nucleic acids.

9. The method of any one of the preceding claims, wherein the APS are coupled by annealing to a splint molecule.

10. The method of any one of the preceding claims, wherein the APS are coupled using Click chemistry.

## Patentansprüche

1. Verfahren zum Erleichtern der Nukleinsäuresequenzassemblierung, wobei das Verfahren Folgendes umfasst:
a) Bereitstellen einer Nukleinsäureprobe, die einzelsträngige Matrizennukleinsäuren umfasst;
b) Priming jeder Matrizennukleinsäure mit einem oder mehreren Strichcodeinitiationsprimern, um annealte Nukleinsäureassemblierungen zu erzeugen, wobei jeder Strichcodeinitiationsprimer einen 3'-Matrizenhybridisierungsabschnitt und einen 5'-Strichcodeinitiationsabschnitt umfasst;
c) Durchführen einer Polymeraseverlängerung, um matrizenkomplementäre Stränge zu erzeugen, die an die Matrizennukleinsäuren annealt bleiben;
d) Durchführen von zwei oder mehr Runden von Split-Pool-Synthese zur Kopplung von Oligonukleotid-Codierungseinheiten "analysierbare Polymeruntereinheiten" (APS), um eine Oligonukleotid-Strichcodesequenz an jeden der matrizenkomplementären Stränge zu assemblieren, die die Strichcoderegion, die Primerregion und die zielspezifische Region, die komplementär ist zu der Zielnukleinsäure, umfassen;
e) Sequenzieren der strichcodierten komplementären Stränge unter Erhalt von Matrizen-DNA-Sequenzlesungen; und
f) Identifizieren einer Strichcodesequenz, die mit jeder Matrizen-DNA-Sequenzlesung in Verbindung steht, um jene Sequenzen zu identifizieren, die aus derselben Matrizennukleinsäure entstanden sind, und Assemblieren der Matrizennukleinsäuresequenz aus den matrizenkomplementären Regionen der strichcodierten komplementären Stränge.

2. Verfahren nach Anspruch 1, ferner umfassend End-Markieren der einzelsträngigen Matrizennukleinsäuren am 3'-Ende mit einem Affinitäts-Tag vor Schritt (b).

3. Verfahren nach Anspruch 1 oder Anspruch 2, wobei das Affinitäts-Tag Biotin ist.

4. Verfahren nach einem der vorstehenden Ansprüche, wobei der 3'-Strichcodeinitiationsabschnitt eine Zufallssequenz umfasst.

5. Verfahren nach einem der vorstehenden Ansprüche, wobei die Polymeraseverlängerung in Schritt c) ein Didesoxynukleotid umfasst.

6. Verfahren nach einem der vorstehenden Ansprüche, wobei die APS Fehlerprüfsubcodes (SC) einschließen, die eine definierte Sequenz von etwa 7 Nukleotiden umfassen, die sich von anderen SC in der Probe um mindestens 3 Nukleotide unterscheidet.

7. Verfahren nach einem der vorstehenden Ansprüche, wobei jede APS eine Zufallssequenz umfasst.

8. Verfahren nach einem der vorstehenden Ansprüche, ferner umfassend einen Schritt des Zählens der strichcodierten komplementären Stränge, die aus derselben Matrizennukleinsäure entstanden sind, wodurch die anfängliche Anzahl von Kopien der Matrizennukleinsäuren nachgewiesen wird.

9. Verfahren nach einem der vorstehenden Ansprüche, wobei die APS durch Annealing an ein Splint-Molekül gekoppelt werden.

10. Verfahren nach einem der vorstehenden Ansprüche, wobei die APS unter Anwendung von Click-Chemie gekoppelt werden.

## Revendications

1. Procédé pour faciliter l'assemblage de séquences d'acide nucléique, le procédé comprenant :
a) la fourniture d'un échantillon d'acide nucléique comprenant des acides nucléiques de matrice monobrins ;
b) l'amorçage de chaque acide nucléique de matrice avec une ou plusieurs amorces d'initiation à code-barres pour créer des assemblages d'acides nucléiques recombinés, dans lequel chaque amorce d'initiation à code-barres comprend une partie d'hybridation à la matrice 3', et une partie d'initiation à code-barres 5' ;
c) la réalisation d'une extension par polymérase pour créer des brins complémentaires de matrice qui restent recombinés aux acides nucléiques de matrice ;
d) la réalisation de deux cycles ou plus de synthèse split-pool couplant des unités de codage oligonucléotidiques « sous-unités polymères dosables » (APS) pour assembler une séquence à code-barres oligonucléotidique sur chacun des brins complémentaires de matrice comprenant la région à code-barres, la région d'amorce et la région spécifique à la cible complémentaire de l'acide nucléique cible ;
e) le séquençage des brins complémentaires à code-barres pour obtenir des lectures de séquence d'ADN de matrice ; et
f) l'identification d'une séquence à code-barres associée à chaque lecture de séquence d'ADN de matrice pour identifier les séquences qui proviennent du même acide nucléique de matrice et l'assemblage de la séquence d'acide nucléique de matrice provenant des régions complémentaires de matrice des brins complémentaires à code-barres.

2. Procédé selon la revendication 1, comprenant en outre le marquage d'extrémité des acides nucléiques de matrice monobrins au niveau de l'extrémité 3' avec un marqueur d'affinité avant l'étape (b).

3. Procédé selon la revendication 1 ou la revendication 2, dans lequel le marqueur d'affinité est la biotine.

4. Procédé selon l'une quelconque des revendications précédentes, dans lequel la partie d'initiation à code-barres 3' comprend une séquence aléatoire.

5. Procédé selon l'une quelconque des revendications précédentes, dans lequel l'extension par polymérase de l'étape c) comprend un didésoxynucléotide.

6. Procédé selon l'une quelconque des revendications précédentes, dans lequel les APS incluent des sous-codes (SC) de vérification d'erreur comprenant une séquence définie d'environ 7 nucléotides qui diffère des autres SC de l'échantillon par au moins 3 nucléotides.

7. Procédé selon l'une quelconque des revendications précédentes, dans lequel chaque APS comprend une séquence aléatoire.

8. Procédé selon l'une quelconque des revendications précédentes, comprenant en outre une étape de comptage des brins complémentaires à code-barres qui proviennent du même acide nucléique de matrice détectant ainsi le nombre initial de copies des acides nucléiques de matrice.

9. Procédé selon l'une quelconque des revendications précédentes, dans lequel les APS sont couplées par recombinaison à une molécule attelle.

10. Procédé selon l'une quelconque des revendications précédentes, dans lequel les APS sont couplées par chimie clic.
